(19) Europäisches Patentamt
European Patent Office
Office européen des brevets

(11) **EP 3 781 231 B1**

(12) **EUROPÄISCHE PATENTSCHRIFT**

(45) Veröffentlichungstag und Bekanntmachung des Hinweises auf die Patenterteilung:
**31.05.2023 Patentblatt 2023/22**

(21) Anmeldenummer: **19721214.5**

(22) Anmeldetag: **16.04.2019**

(51) Internationale Patentklassifikation (IPC):
***A61M 1/36*** *(2006.01)*

(52) Gemeinsame Patentklassifikation (CPC):
**A61M 1/3627; A61M 1/3641;** A61M 2205/3331

(86) Internationale Anmeldenummer:
**PCT/EP2019/059835**

(87) Internationale Veröffentlichungsnummer:
**WO 2019/201943 (24.10.2019 Gazette 2019/43)**

(54) **DRUCKMESSLEITUNG UND IHRE VERWENDUNG**

PRESSURE MEASUREMENT LINE AND USE THEREOF

TUYAU DE MESURE DE PRESSION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.04.2018 DE 102018109563**

(43) Veröffentlichungstag der Anmeldung:
**24.02.2021 Patentblatt 2021/08**

(73) Patentinhaber: **Fresenius Medical Care Deutschland GmbH**
**61352 Bad Homburg (DE)**

(72) Erfinder:
• **KLEWINGHAUS, Juergen**
**61440 Oberursel (DE)**
• **SCHROERS, Alexander**
**60389 Frankfurt (DE)**

(74) Vertreter: **Bobbert & Partner**
**Patentanwälte PartmbB**
**Postfach 1252**
**85422 Erding (DE)**

(56) Entgegenhaltungen:
**DE-A1-102015 102 040**

**Beschreibung**

Die vorliegende Erfindung betrifft eine Druckmessleitung gemäß Anspruch 1. Sie betrifft zudem eine Blutkammer gemäß Anspruch 6 und einen extrakorporalen Blutschlauchsatz gemäß Anspruch 7. Ferner betrifft sie eine Erfassungseinrichtung gemäß Anspruch 9 sowie eine Blutbehandlungsvorrichtung gemäß Anspruch 15.

Aus der Praxis ist bekannt, einen extrakorporalen Blutkreislauf über eine Druckmessleitung, die von dem Blutkreislauf abzweigt, an einen Drucksensor anzuschließen, um den im Blutkreislauf herrschenden Druck zu messen. Der Drucksensor ist regelmäßig Teil einer Behandlungsvorrichtung. Die Druckmessleitung, die zumeist eine Stichleitung ist, ist mit dem Drucksensor verbunden. In ihr ist stets eine bestimmte Menge Luft eingeschlossen, über welche der Druck mittels Drucksensor gemessen wird. Um das unerwünschte Vordringen von Blut bis in die Blutbehandlungsmaschine hinein zu verhindern, ist mindestens ein hydrophober Schutzfilter in der Leitung angeordnet, der zumeist die Form einer Membran hat oder eine solche aufweist. Dieser Hydrophobfilter wird zusammen mit dem restlichen Blutschlauchset des extrakorporalen Blutkreislaufs nach einer erfolgten Blutbehandlung verworfen.

Während des Betriebs des extrakorporalen Blutkreislaufs kann es vorkommen, dass der Hydrophobfilter durch Blut benetzt wird und dadurch zumindest teilweise verstopft. Hieraus resultiert eine Beeinträchtigung der Funktionsfähigkeit der Druckmessung. Soweit der Anwender dieses Problem erkennt, muss er den Hydrophobfilter durch einen neuen Hydrophobfilter ersetzen. Der neue Hydrophobfilter kann ein Ersatzteil sein oder in einem Ersatzteil enthalten sein. Er kann insbesondere eine Ersatzdruckmessleitung sein, die konfiguriert ist, um an einen freien Anschluss der Blutkammer oder des Blasenfängers angeschlossen zu werden. Der gesamte extrakorporale Blutschlauchsatz muss dann nicht ausgewechselt werden.

Aus der DE 10 2011 108 784 A9 sind Verfahren sowie Vorrichtungen zum Überprüfen wenigstens einer Funktion einer medizinischen Funktionseinrichtung bekannt.

Die DE 10 2015 102040 A1 offenbart eine Druckmessleitung mit einer Membran und mit einem Verbinder zum Verbinden mit einer Blutbehandlungsvorrichtung, wobei die Druckmessleitung einen konstanten Durchmesser über ihre gesamte Länge aufweist.

Eine Aufgabe der vorliegenden Erfindung ist es, eine weitere Druckmessleitung vorzuschlagen. Ein Verfahren zur Überwachung einer Druckmessleitung wird offenbart. Zudem sollen geeignete Einrichtungen und Vorrichtungen zum Ausführen des offenbarten Verfahrens angegeben werden. Das hierin offenbarte Verfahren zählt nicht zur Erfindung.

Die erfindungsgemäße Aufgabe wird durch eine Druckmessleitung mit den Merkmalen des Anspruchs 1 gelöst. Sie wird ferner gelöst durch eine Blutkammer mit den Merkmalen des Anspruchs 6 und durch einen extrakorporalen Blutschlauchsatz mit den Merkmalen des Anspruchs 7. Ferner wird sie gelöst durch eine Erfassungseinrichtung mit den Merkmalen des Anspruchs 9 sowie durch eine Blutbehandlungsvorrichtung mit den Merkmalen des Anspruchs 15.

Erfindungsgemäß wird somit eine Druckmessleitung mit einer Membran und mit einem Verbinder zum Verbinden der Druckmessleitung mit einer Blutbehandlungsvorrichtung zur Blutbehandlung vorgeschlagen, wobei letztere optional einen Druckluftausgang aufweist. Der Verbinder ist optional zum Verbinden der Druckmessleitung mit einem Ausgang der Blutbehandlungsvorrichtung ausgestaltet, vorzugsweise mit dem Druckluftausgang.

Erfindungsgemäß weist die Druckmessleitung zusätzlich wenigstens zwei aufeinander folgende Lumenabschnitte auf, nämlich einen ersten Lumenabschnitt und einen zweiten Lumenabschnitt. Der erste Lumenabschnitt weist hierbei eine erste Lumengeometrie auf. Der zweite Lumenabschnitt weist eine zweite Lumengeometrie auf. Die erste und die zweite Lumengeometrie unterscheiden sich zumindest in ihrem Durchmesser oder nur in ihrem Durchmesser voneinander.

Die erfindungsgemäße Blutkammer umfasst eine erfindungsgemäße Druckmessleitung oder ist hiermit verbunden. Die Blutkammer kann beispielsweise konfiguriert sein, um in einer Blutentnahmeleitung und/oder in einer Blutrückgabeleitung eines extrakorporalen Blutkreislaufs eingebaut zu sein oder zu werden.

Die erfindungsgemäße Blutkammer kann eine arterielle und/oder venöse Blutkammer sein.

Der erfindungsgemäße extrakorporale Blutschlauchsatz weist wenigstens eine erfindungsgemäße Blutkammer und/oder eine erfindungsgemäße Druckmessleitung auf oder ist mit wenigstens einer dieser beiden Einrichtungen verbunden.

Erfindungsgemäß wird eine Erfassungseinrichtung vorgeschlagen, welche zum Durchführen oder Veranlassen eines Verfahrens zum Überwachen einer Druckmessleitung eines extrakorporalen Blutschlauchsatzes programmiert und/oder konfiguriert ist.

Das hierin offenbarte Verfahren läuft ab, wenn eine Blutbehandlungsvorrichtung mittels einer Verbindungsstelle mit einer Druckmessleitung eines extrakorporalen Blutschlauchsatzes verbunden ist. Des Weiteren ist die Behandlungsvorrichtung mit einem Drucksensor ausgestaltet, welcher zum Messen des in der Druckmessleitung herrschenden Drucks angeordnet ist.

In weiteren Schritten des offenbarten Verfahrens erfolgt ein Messen eines in der Druckmessleitung herrschenden Drucks oder einer Druckveränderung über der Zeit mittels des Drucksensors sowie ein Ermitteln einer Abweichungsgröße (alternativ: "Abweichungsmaß") des Drucks oder deren Veränderung.

Ferner erfolgt ein Auswerten der Abweichungsgröße des gemessenen Drucks, oder der Veränderung derselben über der Zeit, jeweils mittels eines Vergleichs der ermittelten Abweichungsgröße oder deren Veränderung mit zuvor gemessenen oder gespeicherten Werten, Schwellenwerten, Bereichen oder Verläufen hierfür.

Dabei kann die Abweichungsgröße als Quadrat der Standardabweichung als ein Maß für die Variabilität des Drucks verstanden werden.

Allgemein kann die Abweichungsgröße (oder "Abweichungsmaß") als Maß für die Variabilität des Drucks verstanden werden und/oder als ein Maß dafür, wie sehr ein gemessener Druckwert von einem Erwartungswert oder erwarteten Wert, etwa einem Mittelwert, aus vergangenen Messungen abweicht. Das Maß der Abweichung kann als mathematische Größe zu einer Aussage führen ("zu groß", "innerhalb vorgegebener Grenzen", usw.) oder hierfür verwendet werden.

Die erfindungsgemäße Blutbehandlungsvorrichtung weist wenigstens einen erfindungsgemäßen extrakorporalen Blutschlauchsatz und/oder eine erfindungsgemäße Erfassungseinrichtung auf oder ist wenigstens mit wenigstens einer dieser beiden Einrichtungen verbunden.

Das Verfahren dient dem Überwachen einer Druckmessleitung eines extrakorporalen Blutschlauchsatzes.

Das Verfahren umfasst ein Bereitstellen einer Blutbehandlungsvorrichtung mit einer Verbindungsstelle, welche ausgestaltet ist, um mit einer Druckmessleitung eines extrakorporalen Blutschlauchsatzes verbindbar zu sein. Des Weiteren ist die Blutbehandlungsvorrichtung mit einem Drucksensor ausgestaltet, welcher zum Messen des in der Druckmessleitung herrschenden Drucks angeordnet ist.

In weiteren Schritten des Verfahrens erfolgt ein Messen eines in der Druckmessleitung herrschenden Drucks oder einer Druckveränderung über der Zeit mittels des Drucksensors und ein Ermitteln einer Abweichungsgröße des Drucks oder deren Veränderung.

Ferner erfolgt ein Auswerten der Abweichungsgröße des gemessenen Drucks oder der Veränderung der Abweichungsgröße über der Zeit, jeweils mittels eines Vergleichs der ermittelten Abweichungsgröße oder deren Veränderung mit zuvor gemessenen oder gespeicherten Werten, Schwellenwerten, Bereichen oder Verläufen.

Ein offenbartes digitales, insbesondere nichtflüchtiges, Speichermedium, insbesondere in Form eines maschinenlesbaren Trägers, insbesondere in Form einer Diskette, CD, DVD oder in Form eines USB-Sticks oder EPROM, insbesondere mit elektronisch oder optisch auslesbaren Steuersignalen, kann derart mit einem programmierbaren Computersystem zusammenwirken, dass die maschinellen Schritte eines hierin offenbarten Verfahrens veranlasst werden.

Ein offenbartes Computerprogramm-Produkt weist einen volatilen, flüchtigen oder auf einem maschinenlesbaren Träger gespeicherten Programmcode oder eine Signalwelle zur Veranlassung der maschinellen Schritte des offenbarten Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Server-System, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein offenbartes Computerprogramm weist einen Programmcode auf zur Veranlassung der maschinellen Schritte des hierin offenbarten Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das offenbarte digitale Speichermedium, das offenbarte Computerprogramm-Produkt und das offenbarte Computerprogramm gilt, dass alle, einige oder manche der maschinell durchgeführten Schritte des offenbarten Verfahrens veranlasst werden. Dies gilt insbesondere im Zusammenwirken mit einer erfindungsgemäßen Erfassungseinrichtung und/oder einer bereitgestellten Blutbehandlungsvorrichtung wie hierin beschrieben, insbesondere an einer Blutbehandlungsvorrichtung, die beim offenbarten Verfahren zum Einsatz kommt.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt dies eine erfindungsgemäße, beispielhafte Ausführungsform dar.

Wenn hierin von "programmiert" oder "konfiguriert" die Rede ist, so ist auch offenbart, diese Begriffe gegeneinander auszutauschen.

Wann immer hierin ein Verfahrensschritt genannt ist, umfasst die vorliegende Erfindung auch eine entsprechende Programmierung oder Konfigurierung einer geeigneten Vorrichtung oder eines Abschnitts hiervon.

Der Begriff "Hydrophobfilter" wird in manchen Ausführungsformen synonym zu einem Abschnitt einer Druckmessleitung gebraucht, welcher eine Membran aufweist oder ist, und umgekehrt. Diese Begriffe können daher hierin gegeneinander ausgetauscht werden. Die Membran kann hydrophob sein, insbesondere luftdurchlässig und flüssigkeitsundurchlässig.

Der hierin genannte Blasenfänger kann ein arterieller und/oder ein venöser Blasenfänger sein.

In beliebigen Ausführungsformen gilt das hierin zur Abweichungsgröße Gesagte alternativ für einen beliebigen Streuungsparameter, etwa für die Varianz, für die Standardabweichung oder die empirische Standardabweichung. In solchen erfindungsgemäßen Ausführungsformen ist die Abweichungsgröße eine Varianz, ein Streuungsparameter, eine Standardabweichung oder dergleichen. Diese Begriffe und Gegenstände sind ebenfalls von der vorliegenden Erfindung umfasst.

In einigen Ausführungsformen ist die Abweichungsgröße definiert als die mittlere quadratische Abweichung des aktuellen Druckwerts von einem gemittelten Druckwert. Der gemittelte Druckwert kann der Mittelwert der zuletzt über die Druckmessleitung gemessenen Druckwerte sein, z. B. die letzten fünf Werte, oder alle Werte, die innerhalb der vergangenen vorbestimmten Zeitdauer von z. B. 0,5 Sekunden, 1 Sekunde, 1,5 Sekunden, usw. gemessen wurden.

In einigen Ausführungsformen kann die Abweichungsgröße in Analogie zur Berechnung der Varianz in der Stochastik berechnet werden:

$$\delta^2 = \frac{1}{n}\sum_{i=1,n}((x_i - x_m)^2) \quad ,$$

mit

n = vorgegebene Anzahl der Messwerte, z. B. 5

i = Laufindex über alle Messwerte

$x_i$ = i-ter Messwert

$x_m$ = Mittelwert aus allen n Messwerten

$\delta^2$ = Abweichungsgröße oder Abweichungsmaß

Beispielsweise kann an einer Dialysemaschine alle 100 ms ein Druckmesswert für den venösen Druck aufgenommen und gespeichert werden. Das Betrachtungszeitintervall zur Analyse des Drucksignals betrage in diesem Beispiel 0,5 s, womit bereits ein charakteristischer Abschnitt des Druckverlaufs erfasst werden kann. Daraus ergibt sich im Beispiel die vorgegebene Anzahl n zu 5 (n = 5) Messwerten für das Betrachtungszeitintervall oder im Betrachtungsintervall. Es werden also 5 Messwerte aufgenommen, und der Mittelwert $x_m$ aller 5 Messwerte wird berechnet. Dann wird unter Verwendung der 5 (Einzel)Messwerte und des Mittelwerts $x_m$ das Abweichungsmaß berechnet.

Optional ist der Berechnungsansatz so formuliert, dass die Abweichung überhöht dargestellt wird. Dies ist in einer Ausführungsform durch den quadratischen Ansatz der Fall, ebenso nach einer mathematischen Operation, in welcher die Abweichung mit einem Exponenten größer als 2 (zwei) oder mit einem ausreichend großen Multiplikator berücksichtigt wird.

In einigen Ausführungsformen wird beim Messen eines in der Druckmessleitung herrschenden Drucks oder einer Druckveränderung über der Zeit mittels des Drucksensors stets der Spitzenwert des Drucksignals gemessen. Schwankt der Druck z. B. periodisch, etwa bedingt durch Druckeinflüsse, die von der Bewegung des Rotors einer als Rollenpumpe ausgestalteten Blutpumpe herrühren, so kann der Druck wiederholend ansteigen und wieder abfallen, sich also zwischen einem oberen Maximalwert und einem unteren Minimalwert bewegen.

Die Präzision der Druckmessung kann dadurch gesteigert werden, dass erfindungsgemäß stets Maximalwerte oder stets Minimalwerte betrachtet werden, etwa beim Ermitteln eines Mittelwerts aus den vergangenen Druckmessungen, und/oder beim Messen des konkreten Druckwerts.

Statt Maximalwerten oder Minimalwerten können Druckmittenwerte verwendet werden, die jeweils zwischen dem Maximal- und dem Minimalwert liegen. Auch aus diesen Druckmittenwerten des einzelnen Drucksignals kann der hierin genannte Mittelwert als Erwartungswert berechnet werden.

Ferner kann erfindungsgemäß die Rotorstellung einzelner Pumpen, z. B. der Blutpumpe, bei der Messung des konkreten Druckwerts in Betracht gezogen werden. So kann vorgesehen sein, den Druck stets dann zu messen, wenn die Blutpumpe (erneut) eine bestimmte Rotorstellung einnimmt. Letzteres kann durch Einsatz eines entsprechenden Sensors, z. B. eines Magnetfeldsensors oder eines Hall-Sensors, und die Auswertung seiner Messungen, sichergestellt werden.

In manchen Ausführungsformen umfasst die erfindungsgemäße Druckmessleitung mehrere Lumenabschnitte mit jeweils einer ersten Lumengeometrie und/oder mehrere Lumenabschnitte mit jeweils einer zweiten Lumengeometrie.

In einigen Ausführungsformen weist die erfindungsgemäße Druckmessleitung getrennt voneinander vorliegende Lumenabschnitte mit jeweils der ersten Lumengeometrie auf.

In manchen Ausführungsformen weist die erfindungsgemäße Druckmessleitung getrennt voneinander vorliegende Lumenabschnitte mit jeweils der zweiten Lumengeometrie auf.

In einigen Ausführungsformen können sich Lumenabschnitte mit der ersten Lumengeometrie mit Lumenabschnitten mit der zweiten Lumengeometrie abwechseln. Durch die unterschiedlichen Lumengeometrien, welche in der Druckmessleitung vorgesehen sind, steigt der Flüssigkeitspegel in den unterschiedlichen Lumenabschnitten unterschiedlich schnell. Die kontinuierliche Abhängigkeit zwischen Füllhöhe und Volumen ist damit aufgehoben und kann der Definition von Schwellwerten für eine zu ermittelnde Abweichungsgröße dienen, mit welcher wiederum die Füllhöhe der Druckmessleitung ermittelt werden kann.

In manchen Ausführungsformen weist die Druckmessleitung einen weiteren Verbinder auf, der zum Verbinden der Druckmessleitung mit einer, insbesondere venösen, Kammer ausgestaltet ist. Diese Kammer kann eine Blutkammer sein.

In gewissen Ausführungsformen sind die Lumenabschnitte mit erster und/oder zweiter Lumengeometrie nicht Teil eines Verbinders der Druckmessleitung und/oder nicht mit einem solchen Verbinder direkt verbunden.

In einigen Ausführungsformen ist die Druckmessleitung des extrakorporalen Blutschlauchsatzes mit der, insbesondere venösen, Blutkammer verbunden.

In gewissen Ausführungsformen der Erfassungseinrichtung ist oder umfasst das Auswerten durch das offenbarte Verfahren das Treffen einer Aussage über die Druckmessleitung.

In manchen Ausführungsformen umfasst das Verfahren das Bereitstellen einer abgeschlossenen Sammlung an Mindestwerten, Maximalwerten, Mustern, Kennlinien und/oder Verläufen von Abweichungsgrößen oder Veränderungen der Abweichungsgröße über der Zeit, welche für eine oder für mehrere vorbestimmte, voneinander verschiedene Druckmessleitungen erhoben wurde oder diese kennzeichnen. Ferner ist oder umfasst das Auswerten ein Überprüfen, ob die Abweichungsgröße oder ihr Verlauf in der abgeschlossenen Sammlung enthalten ist.

In einigen Ausführungsformen der Erfassungseinrichtung ergibt oder ist die getroffene Aussage bei der Auswertung des hierin offenbarten Verfahrens, dass die Gefahr einer Benetzung der Membran und/oder des Sensors besteht und/oder, dass ein Flüssigkeitspegel in der Druckmessleitung unzulässig ansteigt und/oder eine vorbestimmte Höhe erreicht oder alternativ überstiegen hat.

In manchen Ausführungsformen der Erfassungseinrichtung ergeht nach einer getroffenen Aussage, dass die Gefahr einer Benetzung der Membran und/oder des Sensors besteht, oder, dass ein Flüssigkeitspegel in der Druckmessleitung unzulässig ansteigt oder angestiegen ist, eine Fehlermeldung oder ein Alarm. Die getroffene Aussage kann hierbei auch eine Unterbrechung oder einen Abbruch eines laufenden Behandlungsverfahrens zur Folge haben oder eine Unterbrechung oder einen Abbruch initiieren, beispielsweise durch einen oder nach einem Hinweis an den Anwender.

In einigen Ausführungsformen umfasst das Verfahren der erfindungsgemäßen Erfassungseinrichtung als weiterer Schritt ein Unterbrechen oder Beenden einer (laufenden) Blutbehandlung mit der Blutbehandlungsvorrichtung. Das Unterbrechen oder Beenden erfolgt in diesen Ausführungsformen, falls das Treffen einer Aussage über die Verbindung ergibt, dass eine Gefahr der Benetzung der Membran oder des Sensors besteht, eine vorbestimmte Höhe des Pegels überschritten ist, oder dass die Abweichungsgröße oder ihr Verlauf nicht in der abgeschlossenen Sammlung enthalten ist.

In manchen Ausführungsformen umfasst das Verfahren der erfindungsgemäßen Erfassungseinrichtung als weiterer Schritt, nach einer getroffenen Aussage über die Gefahr einer Benetzung der Membran und/oder des Sensors, oder, dass der Flüssigkeitspegel in der Druckmessleitung unzulässig ansteigt oder angestiegen ist, das Ergreifen von Maßnahmen mittels der Blutbehandlungsvorrichtung, um den Flüssigkeitspegel in der Druckmessleitung und/oder in der, insbesondere venösen, Blutkammer oder dem, insbesondere venösen, Blasenfänger zu senken, welche(r) mit der Druckmessleitung, insbesondere unmittelbar, in Fluidverbindung verbunden ist.

In manchen Ausführungsformen umfassen die Maßnahmen mittels der Blutbehandlungsvorrichtung, um den Flüssigkeitspegel im Blasenfänger zu senken, ein Verdrängen eines Teils der Flüssigkeit mittels Gas, z. B. mittels Luft aus der Drucklufteinrichtung oder -quelle, oder bestehen hieraus.

Diese Maßnahme kann von der Erfassungseinrichtung oder der Blutbehandlungsvorrichtung automatisch eingeleitet werden. Damit kann erreicht werden, dass der Pegel in der Druckmessleitung gesenkt wird, bevor die Membran in Gefahr gerät, benetzt zu werden.

Andere Maßnahmen wie Fehlermeldung, Alarm, Unterbrechen der Behandlung und dergleichen können alternativ oder ergänzend ebenfalls vorgesehen sein und optional automatisiert, etwa mittels der Erfassungseinrichtung oder der Blutbehandlungsvorrichtung, ablaufen.

In einigen Ausführungsformen umfasst das hierin offenbarte Verfahren ein Sperren von manchen oder allen Behandlungsmodalitäten der bereitgestellten Blutbehandlungsvorrichtung und/oder Einschränken von Behandlungsparametern von mittels der bereitgestellten Blutbehandlungsvorrichtung durchführbaren Blutbehandlungsverfahren. Das Sperren erfolgt in diesen Ausführungsformen, falls das Treffen einer Aussage über die Verbindung ergibt, dass eine Benetzung der Membran oder des Sensors besteht, eine vorbestimmte Höhe des Pegels überschritten ist und/oder, dass die Abweichungsgröße und/oder ihr Verlauf nicht in der abgeschlossenen Sammlung enthalten ist.

In manchen Ausführungsformen ist die Blutbehandlungsvorrichtung der Erfassungseinrichtung als Vorrichtung zur Apherese oder Dialyse, wiederum insbesondere zur Hämodialyse, Hämofiltration oder Hämodiafiltration ausgestaltet.

In einigen Ausführungsformen weist die Erfassungseinrichtung wenigstens eine Anzeigeeinrichtung zum Anzeigen eines Ergebnisses der Durchführung des offenbarten Verfahrens auf oder ist hiermit in Signalkommunikation verbunden. Die Erfassungseinrichtung ist programmiert zum Ausgeben entsprechender Signale zum Anzeigen des Ergebnisses an die Anzeigeeinrichtung. Die Anzeigeeinrichtung kann ein Display, eine Fehleranzeige oder dergleichen sein.

In manchen Ausführungsformen ist die Blutbehandlungsvorrichtung als Vorrichtung zur Apherese oder Dialyse, wiederum insbesondere zur Hämodialyse, Hämofiltration oder Hämodiafiltration ausgestaltet.

In einigen erfindungsgemäßen, beispielhaften Ausführungsformen weist die Erfassungseinrichtung wenigstens eine Alarmeinrichtung auf, welche konfiguriert oder programmiert ist zum Ausgeben eines Alarms für den Fall, dass das Ergebnis der Durchführung des Verfahrens ist, dass eine Benetzung der Hydrophobmembran droht. Alternativ steht sie mit einer solchen Alarmeinrichtung in Signalübertragung.

In einigen erfindungsgemäßen, beispielhaften Ausführungsformen ist die Erfassungseinrichtung eine Steuerungsvorrichtung und/oder ein Funktionstestmonitor oder weist dergleichen jeweils auf.

In gewissen Ausführungsformen ist oder umfasst das Auswerten des offenbarten Verfahrens das Treffen einer Aussage über die Druckmessleitung.

In manchen Ausführungsformen des offenbarten Verfahrens ist oder ergibt das Treffen einer Aussage, dass die Gefahr einer Benetzung der Membran und/oder des Sensors besteht und/oder ein Flüssigkeitspegel in der Druckmessleitung steigt und/oder eine vorbestimmte Höhe des Pegels erreicht ist.

In manchen Ausführungsformen des offenbarten Verfahrens ergeht nach einer getroffenen Aussage, dass die Gefahr einer Benetzung der Membran und/oder des Sensors besteht, oder, dass ein Flüssigkeitspegel in der Druckmessleitung unzulässig ansteigt oder angestiegen ist, eine Fehlermeldung oder ein Alarm. Die getroffene Aussage kann hierbei auch eine Unterbrechung oder einen Abbruch eines laufenden Behandlungsverfahrens zur Folge haben oder eine Unterbrechung oder einen Abbruch initiieren, beispielsweise durch einen oder verbunden mit einem Hinweis an den Anwender.

In einigen Ausführungsformen des offenbarten Verfahrens wird als weiterer Schritt ein Unterbrechen einer (laufenden) Blutbehandlung mit einer Blutbehandlungsvorrichtung umfasst. Dies kann insbesondere für eine solche Blutbehandlung gelten, bei welcher der extrakorporale Blutschlauchsatz bestimmungsgemäß verwendet wird oder verwendet werden soll. Das Unterbrechen oder Beenden erfolgt in diesen Ausführungsformen insbesondere, falls das Treffen einer Aussage über die Verbindung ergibt, dass eine Gefahr einer Benetzung oder eine Benetzung der Membran oder des Sensors besteht, dass ein Flüssigkeitspegel in der Druckmessleitung unzulässig ansteigt oder angestiegen ist, oder, dass die Abweichungsgröße oder ihr Verlauf nicht in der abgeschlossenen Sammlung enthalten ist.

Das Ergreifen von Maßnahmen zum Senken des Flüssigkeitsspiegels im Blasenfänger mittels der Blutbehandlungsvorrichtung kann in gewissen Ausführungsformen des hierin offenbarten Verfahrens ebenso als weiterer Schritt umfasst sein.

In manchen Ausführungsformen des offenbarten Verfahrens ist die Blutbehandlungsvorrichtung als Vorrichtung zur Apherese oder Dialyse, wiederum insbesondere zur Hämodialyse, Hämofiltration oder Hämodiafiltration ausgestaltet.

In manchen Ausführungsformen wird mittels eines Kompressors der Luftdruck in der Druckmessleitung und folglich auch in der, insbesondere venösen, Blutkammer derart erhöht und die, insbesondere venöse, Klemme geöffnet, dass der Flüssigkeitspegel sinkt und damit eine Benetzung der Membran und/oder des Drucksensors vermieden wird. Alternativ oder ergänzend kann eine laufende Blutbehandlung unterbrochen werden und ein Anwender aufgefordert werden, die Fortsetzung der Behandlung freizugeben.

In manchen Ausführungsformen umfasst das offenbarte Verfahren kein Freiblasen der Membran eines Hydrophobfilters mittels einer mit ihm verbundenen Druckluftquelle, um die Funktionsfähigkeit des Filters wiederherzustellen.

In einigen Ausführungsformen endet das hierin offenbarte Verfahren vor einem Feststellen, dass die Membran des Hydrophobfilters verstopft ist.

In manchen Ausführungsformen werden Werte der Abweichungsgröße, die gleich Null sind, oder ausbleibende Schwankungen der Abweichungsgröße nicht zu einer Aussage verwendet, nach welcher Gefahr einer Benetzung der Membran und/oder des Sensors besteht.

In einigen Ausführungsformen werden Amplitudenschwankungen, und insbesondere periodisch wiederkehrende Amplitudenschwankungen, des Drucks nicht erfasst und/oder nicht als Kriterium für die Überwachung der Druckmessleitung oder der Druckmessanordnung verwendet.

In manchen Ausführungsformen umfasst das offenbarte Verfahren nicht, dass aufgrund des Vorhandenseins von Amplitudenschwankungen des Drucks auf die korrekte Funktion der Druckmessleitung oder der Druckmessanordnung geschlossen wird.

In einigen Ausführungsformen weist die Druckmessleitung keine Pumpe, insbesondere Vakuumpumpe, und/oder keinen Abfallbehälter auf oder ist hiermit in Fluidverbindung verbunden. Ganz insbesondere weist die Druckmessleitung eine solche Pumpe nicht zwischen ihrem Verbinder zu ihrem Verbinden mit der Blutbehandlungsvorrichtung und einem Ende, das zu ihrem Verbinden mit einem Blutschlauchsatz, z. B. mit einer Blutkammer hiervon, vorgesehen ist, auf.

In manchen Ausführungsformen ist die Membran nicht zum Herausfiltern von Bestandteilen, insbesondere nicht von festen und/oder flüssigen Bestandteilen, wie etwa Wundsekret, aus einem Fluidstrom geeignet, vorgesehen und/oder eingesetzt.

Einige oder alle erfindungsgemäßen Ausführungsformen können einen oder mehrere der oben oder im Folgenden genannten Vorteile aufweisen.

So kann, da erfindungsgemäß sicherheitsrelevante Funktionen des verwendeten Blutschlauchsatzes, nämlich die Möglichkeit der korrekten Druckmessung, und damit der Blutbehandlungsvorrichtung sichergestellt werden, eine erhöhte Sicherheit für den Patienten geschaffen werden.

Da erfindungsgemäß ein technischer Fehler am Drucksensor (ein Benetzen des Drucksensors und/oder der Hydrophobmembran) bereits erkannt wird, bevor dieser auftritt, vermeidet man einen unnötigen Mehrverbrauch an Einwegartikeln, der beim Austauschen des benetzten Drucksensors und ggf. der zugehörigen Druckmessleitung oder sogar des gesamten extrakorporalen Blutschlauchsatzes anfallen würde.

Es trägt auch dazu bei, Zeit einzusparen, die man andernfalls für das Austauschen eines benetzten Drucksensors und/oder des gesamten Blutschlauchsatzes verwenden müsste.

Eine Befüllung der Druckmessleitung mit Blut und das hierdurch bedingte Benetzen der Membran und/oder des Sensors, was einen notwendigen Austausch der Druckmessleitung nach sich zieht, wird vorteilhaft vermieden. Somit wird ferner auch die Gefahr der Kontamination des Dialysegeräts vermindert.

Durch das hierin offenbarte Verfahren kann auch ein Anstieg von Blut in der Druckmessleitung detektiert werden.

Die vorliegende Erfindung wird im Folgenden anhand der beigefügten Zeichnungen, in welchen identische Bezugszeichen gleiche oder ähnliche Bauteile bezeichnen, exemplarisch erläutert. In den zum Teil stark vereinfachten Figuren gilt:

Fig. 1      zeigt ein schematisch vereinfachtes Schaubild einer erfindungsgemäßen Blutbehandlungsvorrichtung und einer ersten Druckmessleitung eines erfindungsgemäßen Blutschlauchsatzes in einer ersten Ausführungsform;

Fig. 2      zeigt schematisch vereinfacht eine erfindungsgemäße Druckmessleitung in einer zweiten Ausführungsform;

Fig. 3a, 3b      zeigen je ein Diagramm, in welchem der mittels des Drucksensors aus Fig. 1 erfindungsgemäß gemessene Druck über der Zeit gezeigt ist;

Fig. 4      zeigt zwei mittels Drucksensor der Fig. 1 gemessene Verläufe der Abweichungsgröße des im Blasenfänger herrschenden venösen Drucks, einmal bei überwiegend mit Luft gefülltem Blasenfänger und einmal bei überwiegend mit Flüssigkeit gefülltem Blasenfänger, jeweils über der Zeit t in Sekunden [s] bei einem eingestellten Blutfluss von 100 ml/min;

Fig. 5      zeigt in einem vereinfachten Diagramm die Abhängigkeit der Abweichungsgröße vom Luftvolumen in der Druckmessleitung und im Blasenfänger;

Fig. 6      zeigt in einem vereinfachten Diagramm die Abhängigkeit des Luftvolumens vom Flüssigkeitspegel in der Druckmessleitung und im Blasenfänger; und

Fig. 7      zeigt einen diskontinuierlichen Anstieg der Abweichungsgröße in Abhängigkeit vom Flüssigkeitspegel, bedingt durch die Geometrie der Druckmessleitung.

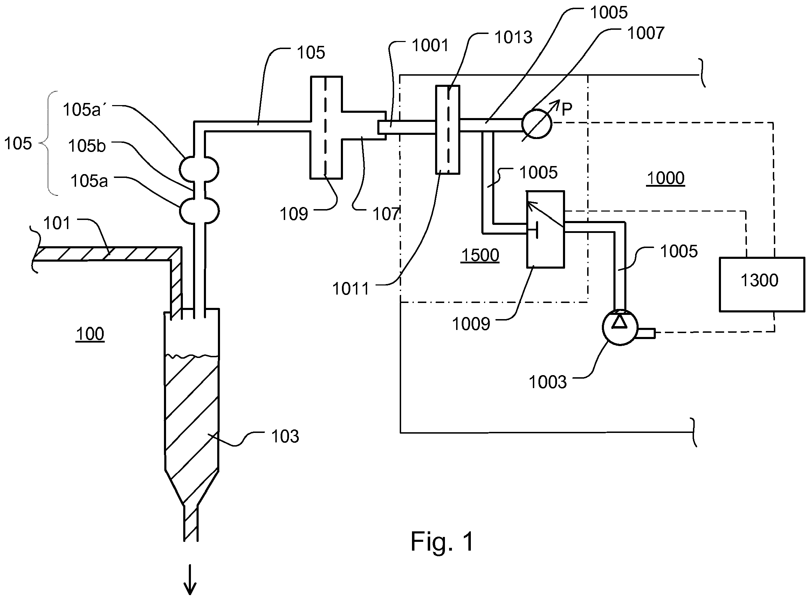

Fig. 1 zeigt schematisch sehr vereinfacht Abschnitte einer erfindungsgemäßen Blutbehandlungsvorrichtung 1000 sowie Abschnitte eines erfindungsgemäßen Blutschlauchsatzes 100.

Die Erfindung ist hierin und insbesondere im Folgenden anhand von erfindungsgemäßen Vorrichtungen erläutert, die auf der venösen Seite des Blutschlauchsatzes angeordnet sind. Die Erfindung ist nicht auf ihren Einsatz auf der venösen Seite eines Blutschlauchsatzes beschränkt.

Der extrakorporale Blutschlauchsatz 100, welcher optional abschnittsweise jeweils außerhalb und innerhalb einer nicht dargestellten Blutkassette verlaufen kann, weist eine venöse Patientenleitung 101 und optional einen venösen Blasenfänger oder eine venöse Blutkammer 103 auf. Eine Durchströmung der Patientenleitung 101 mit Blut erfolgt bei deren Gebrauch zumeist in der Richtung des angegebenen Pfeils in Richtung Patient.

Mit dem Blutschlauchsatz 100 verbunden (oder Teil hiervon) ist eine erfindungsgemäße Druckmessleitung 105. Diese geht in Fig. 1 rein exemplarisch von der venösen Blutkammer 103, hier als Blasenfänger ausgestaltet, ab. Die Druckmessleitung 105 kann die Rücklaufdruckmessleitung sein. Sie kann eine "open line" sein.

Die Druckmessleitung 105 weist optional einen Verbinder 107 auf, welcher zum Verbinden der Druckmessleitung 105 mit beispielsweise einem Druckluftausgang 1001 der Blutbehandlungsvorrichtung 1000 vorgesehen und entsprechend ausgestaltet ist. Rein optional sind der Verbinder 107 und der Druckluftausgang 1001 weibliche bzw. männliche Hälften eines Luer-Konnektors mit weiblichem bzw. männlichem Dichtkonus oder Hälften eines entsprechenden Luer-Lock-Konnektors mit oder ohne zusätzlichem Sicherungsgewinde.

Der Druckluftausgang 1001 kann in oder an einer Außenwand der Blutbehandlungsvorrichtung 1000, beispielsweise in deren Gehäusewand, liegen.

Der Verbinder 107 der Druckmessleitung 105, oder ein anderer Abschnitt der Druckmessleitung 105, welcher in einem im Gebrauchszustand der Druckmessleitung 105 von Luft durchströmten oder von Luft durchströmbaren Bereich der Druckmessleitung 105 liegt, weist eine luftdurchlässige Membran 109 auf. Die luftdurchlässige Membran 109 ist, rein beispielhaft, als hydrophobe Membran oder als Hydrophobfilter ausgestaltet. Die luftdurchlässige Membran 109 ist der fachkundigen Person auch unter der Bezeichnung "transducer protector" bzw. abgekürzt "TP" bekannt.

Die Blutbehandlungsvorrichtung 1000 weist einen Kompressor 1003 als Beispiel für eine Drucklufteinrichtung oder -quelle auf.

Kompressor 1003 und Druckluftausgang 1001 sind beispielsweise mittels einer Druckluftleitung 1005 in Fluidkommunikation verbunden. Der Kompressor 1003 kann optional weitere Ventile für einen anderen als seinen hierin beschriebenen Einsatz aufweisen.

In oder an der Druckluftleitung 1005 sind ein Drucksensor 1007 und, rein exemplarisch, ein optionales Umschaltventil 1009 (alternativ oder ergänzend eine Drossel, ein Schalter, eine Sperre und/oder dergleichen) vorgesehen.

Wie aus Fig. 1 erkannt werden kann, ist der Drucksensor 1007 vorzugsweise derart in die Druckluftleitung 1005 eingebunden oder steht mit dieser in geeigneter Fluidkommunikation, dass er bei entsprechend geschaltetem Umschaltventil 1009 (falls vorhanden) und aktivem, d. h. eingeschaltetem, Kompressor 1003 den aufgrund des Betriebs des Kompressors 1003 in der Druckleitung 1005 herrschenden Druck P messen kann. Der Drucksensor 1007 kann der Rücklaufdrucksensor sein.

Rein beispielhaft führt die Druckluftleitung 1005 durch einen optional vorgesehenen Schutzfilter 1011, welcher wiederum eine, vorzugsweise hydrophobe, im Strömungslauf liegende luftdurchlässige Membran 1013 aufweist.

Die Blutbehandlungsvorrichtung 1000 weist eine Erfassungseinrichtung 1300 auf. Diese ist, wie mittels Strichlinien gezeigt, beispielhaft mit dem Kompressor 1003, dem Drucksensor 1007 und/oder dem Umschaltventil 1009 in Signalkommunikation verbunden.

Wie mittels Strich-Punkt-Linien gezeigt, können wenigstens der Druckluftausgang 1001 und der Drucksensor 1007, ferner optional das Umschaltventil 1009 und der Schutzfilter 1011, soweit vorhanden, Teil der eigenständigen Druckmesseinheit 1500 sein, welche mit der Blutbehandlungsvorrichtung 1000 verbunden ist.

Wie Fig. 1 zu entnehmen ist, weist die Druckmessleitung 105 wenigstens einen ersten Lumenabschnitt 105a und einen hierzu unmittelbar benachbarten zweiten Lumenabschnitt 105b auf.

Der erste Lumenabschnitt 105a weist eine erste Lumengeometrie auf, der zweite Lumenabschnitt 105b eine zweite Lumengeometrie. Die erste und die zweite Lumengeometrie unterscheiden sich zumindest in ihrem Durchmesser voneinander, indem der erste Lumenabschnitt 105a einen größeren Durchmesser aufweist als der zweite Lumenabschnitt 105b. Der erste Lumenabschnitt 105a kann daher als Erweiterung oder Kompartiment bezeichnet werden, der zweite Lumenabschnitt 105b als Engstelle.

Zusätzlich zum ersten Lumenabschnitt 105a weist die Druckmessleitung 105 einen weiteren Lumenabschnitt 105a' auf. Dieser hat ebenfalls einen größeren Durchmesser als der hierzu unmittelbar benachbarte zweite Lumenabschnitt. Er kann dieselbe Geometrie und/oder denselben Durchmesser haben wie der erste Lumenabschnitt 105a. Die Lumenabschnitte 105a und 105a' werden daher hierin gemeinsam als "erste Lumenabschnitte" bezeichnet.

Der extrakorporale Blutkreislauf 100 weist neben den vorstehend beschriebenen Komponenten ferner jeweils optional einen arteriellen Leitungsabschnitt auf, der mit einem nicht dargestellten Patienten und mit einer ebenfalls nicht gezeigten Blutbehandlungseinrichtung, hier exemplarisch ein Blutfilter oder Dialysator, in Fluidverbindung steht. Der Blutfilter weist eine Dialysierflüssigkeitskammer und eine Blutkammer auf, welche durch eine zumeist semi-permeable Membran voneinander getrennt sind.

Die in Fig. 1 nur durch einige ihrer Einrichtungen und schematisch repräsentierte Blutbehandlungsvorrichtung 1000 weist ferner eine Blutpumpe auf. Sie fördert während der Behandlung des Patienten Blut durch Abschnitte des extrakorporalen Blutkreislaufs 100 und in Richtung zum Blutfilter oder Dialysator.

Mittels einer Pumpe für Dialysierflüssigkeit, die als Rollenpumpe oder als anderweitig okkludierende Pumpe ausgestaltet sein kann, wird frische Dialysierflüssigkeit aus einer Quelle entlang einer Dialysierflüssigkeitszulaufleitung in die Dialysierflüssigkeitskammer gepumpt. Die Dialysierflüssigkeit verlässt die Dialysierflüssigkeitskammer als Filtrat in Richtung eines Ausgusses.

Eine weitere Quelle mit Substituat kann optional vorgesehen sein.

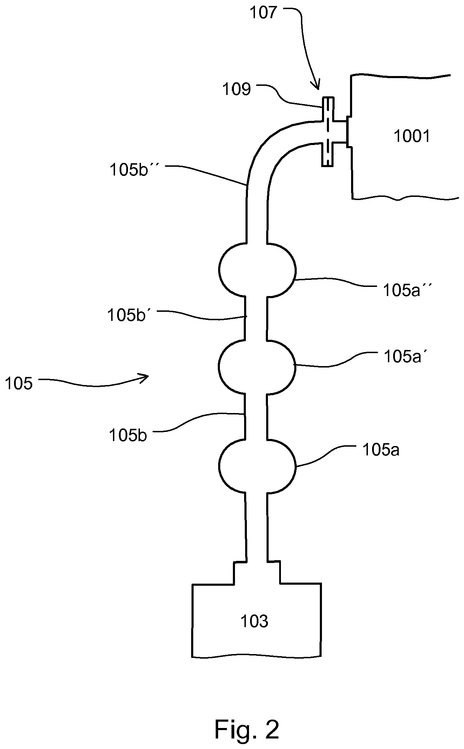

Fig. 2 zeigt eine weitere Ausführungsform der erfindungsgemäßen Druckmessleitung 105. Diese ist mit einem Druckluftausgang 1001 und einem Blasenfänger als Beispiel für eine Blutkammer 103 verbunden, vergleiche hierzu Fig. 1.

Die Druckmessleitung 105 weist mehrere, z. B. drei, erste Lumenabschnitte 105a, 105a' und 105a", also Lumenabschnitte eines ersten Typs, auf. Sie sind jeweils von zweiten Lumenabschnitten 105b, 105b' und 105b" voneinander getrennt, also von Lumenabschnitten eines zweiten Typs. Hier wechseln sich also exemplarisch Lumenabschnitte des ersten Typs mit solchen des zweiten Typs ab.

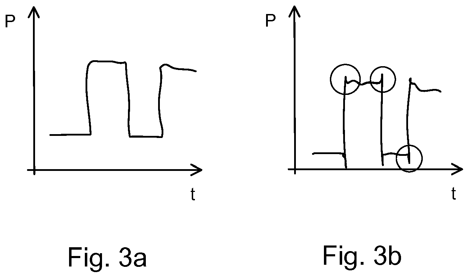

**Fig. 3a und 3b** zeigen je ein Diagramm, in welchem der Verlauf des mittels des Drucksensors 1007 aus Fig. 1 gemessenen Drucks P (z. B. in der Einheit mmHg), über der Zeit t (z. B. in der Einheit s für Sekunden) aufgetragen ist.

Fig. 3a zeigt das mittels Drucksensor 1007 gemessene Drucksignal, wenn die obere Hälfte der Blutkammer 103 mit Luft gefüllt ist.

Fig. 3b zeigt das mittels Drucksensor 1007 gemessene Drucksignal, wenn die Blutkammer 103 bis zu seinem oberen Rand mit Flüssigkeit gefüllt ist, also keine oder kaum Luft aufweist.

Es ist erkennbar, dass die mit Kreisen markierten Überschwinger bei Druckänderungen im (halb-)luftgefüllten System der Fig. 3a deutlich geringer sind als in Fig. 3b.

Dieses Verhalten wird erfindungsgemäß über die Abweichungsgröße, die hier rein exemplarisch als eine quadrierte und ggf. der Varianz entsprechende oder nachempfundene Größe ist und daher exemplarisch mit $\delta^2$ bezeichnet wird, ausgewertet.

Die Abweichungsgröße $\delta^2$ ist hier exemplarisch die mittlere quadratische Abweichung des aktuellen Druckwerts von einem gemittelten Druckwert (z. B. 5 Druckwerte innerhalb von 500 ms).

Die Abweichungsgröße muss jedoch keine quadratische Abweichung sein. Das Beispiel der vorliegenden Figuren ist daher keineswegs beschränkend zu verstehen.

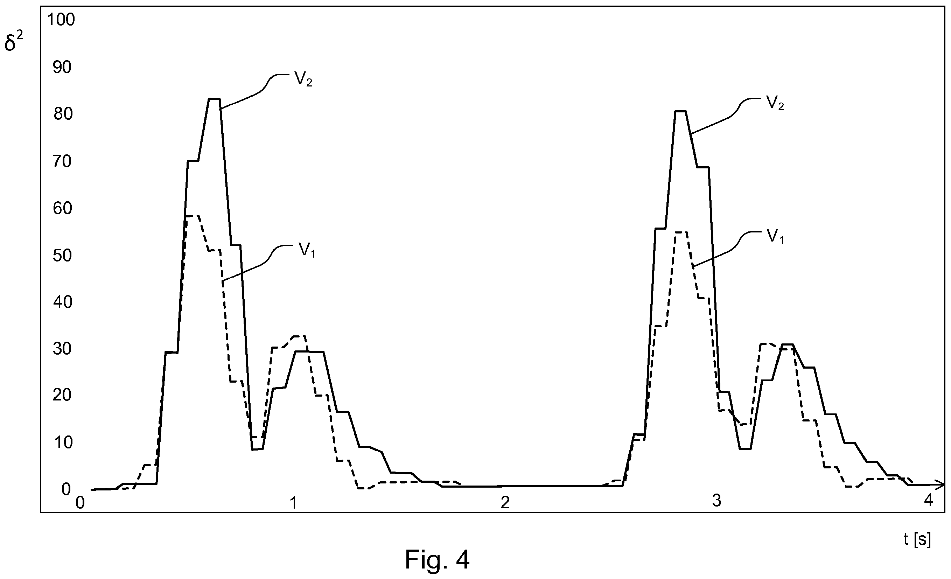

**Fig. 4** zeigt zwei mittels des Drucksensors 1007 der Fig. 1 gemessene Verläufe der Abweichungsgröße $\delta^2$ der Fig. 3 des in der Blutkammer 103 herrschenden venösen Drucks, einmal bei überwiegend mit Luft gefüllter Blutkammer 103 und einmal bei überwiegend mit Flüssigkeit gefüllter Blutkammer 103, jeweils über der Zeit t in Sekunden [s] bei einem eingestellten Blutfluss von 100 ml/min.

Dabei zeigt der Verlauf V1 (gestrichelt) der Abweichungsgröße $\delta^2$ mit Werten zwischen 0 und rund 55 jene Abweichungsgröße $\delta^2$, welche bei Messung des Drucks ermittelt werden kann, wenn sich vergleichsweise viel Luft in der Blutkammer 103 befindet, was eine ungewollte Benetzung des Drucksensors 1007 eher unwahrscheinlich macht.

Der Verlauf V2 (durchgezogene Linie) zeigt hingegen eine Abweichungsgröße $\delta^2$, die Werte zwischen 0 und rund 85 annimmt, als jene Abweichungsgröße $\delta^2$, welche bei Messung des Drucks ermittelt werden kann, wenn sich vergleichsweise wenig Luft in der Blutkammer 103 befindet, was eine ungewollte Benetzung der Membran 109, die wiederum den Drucksensor 1007 davor schützen soll, selber benetzt zu werden, eher wahrscheinlich macht.

Eine Ursache für die unterschiedlichen Verläufe V1 und V2 liegt in der kompressiblen und daher dämpfenden Eigenschaft des Luftvolumens, wobei die dämpfende Wirkung in der Druckmessleitung mit zunehmendem Flüssigkeitspegel und dadurch abnehmendem Luftvolumen abnimmt und die Abweichungsgröße des Drucks zunimmt. Wenn ein Lumenabschnitt mit Flüssigkeit gefüllt ist, dann weist dieser Lumenabschnitt keine dämpfende Wirkung aufgrund von Luft auf und trägt nicht zur gesamten Dämpfung bei.

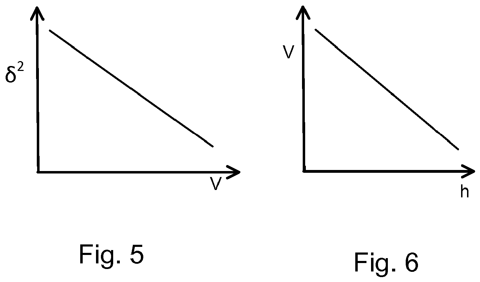

**Fig. 5** zeigt in einem einfachen Diagramm die Abhängigkeit der Abweichungsgröße $\delta^2$ vom Luftvolumen V in der Druckmessleitung 105 und in der Blutkammer 103.

**Fig. 6** zeigt in einem vereinfachten Diagramm die Abhängigkeit des Luftvolumens V vom Flüssigkeitspegel h in der Druckmessleitung 105 unter der Voraussetzung eines konstanten Querschnitts der Druckmessleitung 105.

Die Höhe der Abweichungsgröße $\delta^2$ hängt also erkennbar von der Luftmenge oder dem Luftvolumen V in der Blutkammer 103 und in der Druckmessleitung 105 ab, wie Fig. 5 zeigt. Das Luftvolumen V, das in der Druckmessleitung 105 vorliegt, ist wiederum linear zur Höhe h des Flüssigkeitsspiegels innerhalb der Blutkammer 103, wie Fig. 6 zeigt.

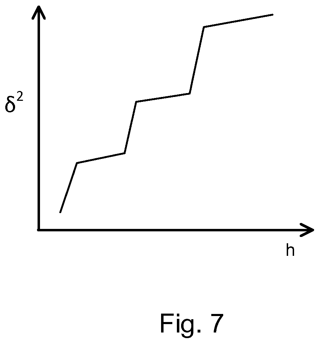

Um genauer bestimmen zu können, bis zu welcher Höhe h in der Druckmessleitung 105 die Flüssigkeit angestiegen ist, ist es wünschenswert, einen diskontinuierlichen Anstieg der Abweichungsgröße $\delta^2$ über der Füllhöhe, also der Höhe h des Flüssigkeitsspiegels, zu erhalten, wie er z. B. in **Fig. 7** gezeigt ist. Sie zeigt die Abweichungsgröße $\delta^2$ in Abhängigkeit vom Flüssigkeitsspiegel in der Druckmessleitung 105 unter der Voraussetzung unterschiedlicher Querschnitte und damit unterschiedlicher Volumina entlang der Druckmessleitung 105.

Zu diesem Zweck wird erfindungsgemäß beispielhaft eine Schlauchgeometrie der Druckmessleitung 105 vorgeschlagen, wie sie z. B. in Fig. 2 gezeigt ist. Die Geometrie weicht von der unveränderlichen Zylinderform bekannter Druckmessleitungen im Längsschnitt ab.

Wenn ein Lumenabschnitt mit Flüssigkeit gefüllt ist, dann weist dieser Lumenabschnitt keine dämpfende Wirkung aufgrund von Luft auf und trägt nicht zur gesamten Dämpfung bei. Bedingt durch die unterschiedlichen Querschnitte und damit Volumina pro Länge der Druckmessleitung steigt der Flüssigkeitspegel in den Abschnitten des ersten Typs (erste Lumengeometrie) langsamer als in jenen des zweiten Typs (zweite Lumengeometrie). Damit ist die kontinuierliche Abhängigkeit zwischen Volumen oder Befüllung einerseits und Füllhöhe andererseits aufgehoben. Somit können vorteilhaft Schwellen für die Abweichungsgröße definiert werden, an welchen abgelesen werden kann, bis wohin oder bis zu welchem Abschnitt (erster Typ oder zweiter Typ) die Flüssigkeit bereits angestiegen ist. Entsprechende Maßnahmen, z. B. wie sie hierin beschrieben sind, können hierauf basierend eingeleitet werden.

Die Länge der Druckmessleitung (vom Verbinder 107) bis zur Blutkammer 103 beträgt exemplarisch zwischen 16,5 bis 27,5 cm; vorzugsweise 22 cm; der Außendurchmesser beträgt beispielsweise 5,5 mm; der Innendurchmesser 3,5 mm; der Werkstoff des Schlauchs ist vorzugsweise PVC (Polyvinylchlorid). Vorzugsweise ist eine dauerhafte Verklebung der Druckmessleitung 105 in einem Stutzen am Verbinder 107 gewählt.

Vorzugsweise ist der Verbinder 107 des freien Endes die weibliche Hälfte eines Luer-Lock-Konnektors mit weiblichem Luer-Konus und umgebendem Außengewinde.

Vorzugsweise beträgt der Durchmesser des kleinsten freien Strömungsquerschnitts im weiblichen Luer-Konus 2,5 mm; der Werkstoff des Luer-Konus ist vorzugsweise PBT (Polybutylenterephthalat).

Die luftdurchlässige Membran 109 hat vorzugsweise einen freien Strömungsdurchmesser im Verbinder 107, der vorzugsweise senkrecht zur Durchströmungsrichtung steht, von 10 bis 14 mm, besonders bevorzugt 12 mm. Die Dicke der luftdurchlässigen Membran 109 beträgt vorzugsweise beispielsweise etwa 0,15 mm.

Der mittlere Porendurchmesser der luftdurchlässigen Membran 109 (deren Werkstoff unter anderen PTFE (Polytetrafluorethylen) sein kann), beträgt vorzugsweise zwischen 0,1 $\mu$m und 0,6 $\mu$m, besonders bevorzugt 0,2 $\mu$m (Mikrometer).

Die Membran 109 ist im Verbinder 107, vorzugsweise beidseitig, optional von strahlenförmigen Stützrippen abgestützt, um die Luftdurchlässigkeit des gesamten freien Querschnitts zu gewährleisten und die Membran gegen erhöhten Druck abzustützen.

Der maschinenseitige Druckluftausgang 1001 kann eine männliche Hälfte des Luer-Lock-Konnektors mit männlichem Luer-Konus und umgebender Überwurfmutter sein. Er kann vorteilhaft hygienisch einwandfrei, da einfach zu reinigen, aus Edelstahl gefertigt sein.

### Bezugszeichenliste

| | |
|---|---|
| 100 | Blutschlauchsatz, Blutkreislauf |
| 101 | venöse Patientenleitung |
| 103 | Blasenfänger, Blasenkammer, Tropfkammer oder, insbesondere venöse, Blutkammer |
| 105 | Druckmessleitung |
| 105a | erster Lumenabschnitt |
| 105a' | erster Lumenabschnitt |
| 105a" | erster Lumenabschnitt |
| 105b | zweiter Lumenabschnitt |
| 105b' | zweiter Lumenabschnitt |
| 105b" | zweiter Lumenabschnitt |
| 107 | Verbinder |
| 109 | luftdurchlässige Membran |
| 1000 | Blutbehandlungsvorrichtung |
| 1001 | Druckluftausgang |
| 1003 | Drucklufteinrichtung, z. B. Kompressor |
| 1005 | Druckluftleitung |
| 1007 | Drucksensor |
| 1009 | Umschaltventil |
| 1011 | Schutzfilter |
| 1013 | luftdurchlässige Membran |
| 1300 | Erfassungseinrichtung |
| 1500 | Druckmesseinheit |
| h | Höhe |
| P | gemessener Druck |
| t | Zeit |
| V | Luftvolumen |

### Patentansprüche

1. Druckmessleitung (105) mit einer Membran (109) und mit einem Verbinder (107) zum Verbinden der Druckmessleitung (105) mit einer Blutbehandlungsvorrichtung (1000), wobei die Druckmessleitung (105) zusätzlich wenigstens zwei aufeinander folgende Lumenabschnitte, nämlich den ersten Lumenabschnitt (105a) und den zweiten Lumenabschnitt (105b), aufweist, wobei der erste Lumenabschnitt (105a) eine erste Lumengeometrie und der zweite Lumenabschnitt (105b) eine zweite Lumengeometrie aufweist, wobei sich die erste Lumengeometrie und die zweite Lumengeometrie zumindest in ihrem Durchmesser voneinander unterscheiden.

2. Druckmessleitung (105) nach Anspruch 1, mit mehreren Lumenabschnitten (105a, 105a', 105a") mit jeweils der ersten Lumengeometrie und/oder mehreren Lumenabschnitten (105b, 105b', 105b") mit jeweils der zweiten Lumengeometrie.

3. Druckmessleitung (105) nach Anspruch 2, wobei Lumenabschnitte (105a, 105a', 105a") mit jeweils der ersten Lumengeometrie getrennt voneinander vorliegen und/oder wobei Lumenabschnitte (105b, 105b', 105b") mit jeweils der zweiten Lumengeometrie getrennt voneinander vorliegen.

4. Druckmessleitung (105) nach einem der Ansprüche 2 bis 3, wobei sich Lumenabschnitte (105a, 105a', 105a") mit jeweils der ersten Lumengeometrie mit Lumenabschnitten (105b, 105b', 105b") mit jeweils der zweiten Lumengeometrie abwechseln.

5. Druckmessleitung (105) nach einem der vorangegangenen Ansprüche mit einem weiteren Verbinder zum Verbinden der Druckmessleitung (105) mit einer Blutkammer (103).

6. Blutkammer (103), aufweisend oder verbunden mit einer Druckmessleitung (105) nach einem der vorangegangenen Ansprüche.

**7.** Extrakorporaler Blutschlauchsatz (100), aufweisend oder verbunden mit wenigstens einer Blutkammer (103) nach Anspruch 6 und/oder einer Druckmessleitung (105) nach einem der Ansprüche 1 bis 5.

**8.** Extrakorporaler Blutschlauchsatz (100) nach Anspruch 7, wobei die Druckmessleitung (105) mit der Blutkammer (103) verbunden ist.

**9.** Erfassungseinrichtung (1300), programmiert und/oder konfiguriert zum Durchführen oder Veranlassen eines Verfahrens zum Überwachen einer Druckmessleitung (105) eines extrakorporalen Blutschlauchsatzes (100), wobei die Druckmessleitung (105) verbunden ist mit einer Blutbehandlungsvorrichtung (1000), wobei die Blutbehandlungsvorrichtung (1000) einen Drucksensor (1007) aufweist, welcher zum Messen des in der Druckmessleitung (105) herrschenden Drucks (P) angeordnet ist, und wobei die Druckmessleitung (105) gemäß einem der Ansprüche 1 bis 5 ausgestaltet ist; wobei das Verfahren die Schritte aufweist:

- Messen eines in der Druckmessleitung (105) herrschenden Drucks (P) oder dessen Veränderung über der Zeit mittels des Drucksensors (1007) und Ermitteln einer Abweichungsgröße ($\delta^2$) des Drucks (P) oder deren Veränderung; und
- Auswerten der Abweichungsgröße ($\delta^2$) oder einer Veränderung der Abweichungsgröße ($\delta^2$) über der Zeit, jeweils mittels eines Vergleichs der ermittelten Abweichungsgröße ($\delta^2$) oder deren Veränderung mit zuvor gemessenen und/oder gespeicherten Werten, Schwellenwerten, Bereichen oder Verläufen hierfür.

**10.** Erfassungseinrichtung (1300) nach Anspruch 9, wobei das Auswerten das Treffen einer Aussage über die Druckmessleitung (105) ist oder umfasst.

**11.** Erfassungseinrichtung (1300) nach einem der Ansprüche 9 bis 10, wobei das Treffen einer Aussage ist oder ergibt, dass die Gefahr einer Benetzung der Membran (109) und/oder des Drucksensors (1007) besteht, eine vorbestimmte Höhe (h) des Flüssigkeitspegels in der Druckmessleitung (105) erreicht ist und/oder dass der Flüssigkeitspegel in der Druckmessleitung (105) steigt.

**12.** Erfassungseinrichtung (1300) nach einem der Ansprüche 9 bis 11, wobei bei Treffen einer Aussage, dass die Gefahr einer Benetzung der Membran (109) besteht, eine Fehlermeldung oder ein Alarm ergeht.

**13.** Erfassungseinrichtung (1300) nach einem der Ansprüche 9 bis 12, mit dem weiteren Schritt:

- Unterbrechen oder Beenden einer Blutbehandlung mittels der Blutbehandlungsvorrichtung (1000);

oder mit dem weiteren Schritt:

- Ergreifen von Maßnahmen mittels der Blutbehandlungsvorrichtung (1000), um den Flüssigkeitsspiegel in der Blutkammer (103) zu senken;

jeweils falls das Treffen einer Aussage über die Verbindung ist oder ergibt, dass die Gefahr einer Benetzung besteht und/oder die vorbestimmte Höhe (h) des Pegels überschritten ist.

**14.** Erfassungseinrichtung (1300) nach Anspruch 9 bis 13, aufweisend wenigstens eine Anzeigeeinrichtung oder hiermit verbunden, und konfiguriert zum Anzeigen eines Ergebnisses der Durchführung des Verfahrens mittels der Anzeigeeinrichtung.

**15.** Blutbehandlungsvorrichtung (1000), aufweisend oder verbunden mit wenigstens einem extrakorporalen Blutschlauchsatz (100) gemäß Anspruch 7 oder 8 und/oder einer Erfassungseinrichtung (1300) gemäß einem der Ansprüche 9 bis 14.

**Claims**

**1.** A pressure measuring line (105) comprising a membrane (109) and a connector (107) for connecting the pressure measuring line (105) to a blood treatment apparatus (1000),
wherein the pressure measuring line (105) additionally comprises at least two consecutive lumen sections, namely the first lumen section (105a) and the second lumen section (105b), the first lumen section (105a) comprising a first lumen geometry and the second lumen section (105b) comprising a second lumen geometry, wherein the first lumen geometry and the second lumen geometry differ from each other at least in their diameter.

**2.** The pressure measuring line (105) according to claim 1, comprising several lumen sections (105a, 105a', 105a") each having the first lumen geometry and/or several lumen sections (105b, 105b', 105b") each having the second lumen geometry.

**3.** The pressure measuring line (105) according to claim 2, wherein lumen sections (105a, 105a', 105a"), each having the first lumen geometry, are separate from each other and/or wherein lumen sections (105b, 105b', 105b"), each having the second lumen geometry, are separate from each other.

4. The pressure measuring line (105) according to anyone of claims 2 to 3, wherein lumen sections (105a, 105a', 105a"), each having the first lumen geometry, alternate with lumen sections (105b, 105b', 105b"), each having the second lumen geometry.

5. The pressure measuring line (105) according to anyone of the preceding claims comprising a further connector for connecting the pressure measuring line (105) to a blood chamber (103).

6. A blood chamber (103) comprising or connected to a pressure measuring line (105) according to anyone of the preceding claims.

7. An extracorporeal blood tubing set (100) comprising or connected to at least one blood chamber (103) according to claim 6 and/or to a pressure measuring line (105) according to anyone of claims 1 to 5.

8. The extracorporeal blood tubing set (100) according to claim 7, wherein the pressure measuring line (105) is connected to the blood chamber (103).

9. A detection device (1300) programmed and/or configured to execute or initiate a method for monitoring a pressure measuring line (105) of an extracorporeal blood tubing set (100), the pressure measuring line (105) being connected to a blood treatment apparatus (1000), wherein the blood treatment apparatus (1000) comprises a pressure sensor (1007) arranged to measure the pressure (P) prevailing in the pressure measuring line (105), and wherein the pressure measuring line (105) is designed according to anyone of claims 1 to 5;
wherein the method comprises the steps of:

- measuring, by means of the pressure sensor (1007), a pressure (P) prevailing in the pressure measuring line (105) or its change over time and determining a deviation variable ($\delta^2$) of the pressure (P) or its change; and
- evaluating the deviation variable ($\delta^2$) or a change in the deviation variable ($\delta^2$) over time, in each case by means of a comparison of the determined deviation variable ($\delta^2$), or its change, with previously measured and/or stored values, threshold values, ranges or courses used therefor.

10. The detection device (1300) according to claim 9, wherein the evaluation is or encompasses reaching a conclusion about the pressure measuring line (105).

11. The detection device (1300) according to anyone of the claims 9 to 10, wherein the reached conclusion is, or results in that a risk of wetting the membrane (109) and/or the pressure sensor (1007) is provided, in that a predetermined height (h) of the liquid level in the pressure measuring line (105) has been achieved and/or the liquid level in the pressure measuring line (105) rises.

12. The detection device (1300) according to anyone of the claims 9 to 11, wherein while the reached conclusion is that a risk of wetting the membrane (109) is provided, an error message or an alarm is triggered.

13. The detection device (1300) according to anyone of the claims 9 to 12 having the further step:

- interrupting or stopping a blood treatment by means of the blood treatment apparatus (1000); or having the further step:

- taking measures, by means of the blood treatment apparatus (1000), to lower the fluid level in the blood chamber (103);

in each case when the reached conclusion about the connection is or results in that a risk of wetting and/or exceeding the predetermined height (h) of the level is provided.

14. The detection device (1300) according to claim 9 to 13, comprising or connected to at least one display device, and configured to display a result of the execution of the method using the display device.

15. A blood treatment apparatus (1000) comprising or connected to at least one extracorporeal blood tubing set (100) according to claim 7 or 8 and/or to a detection device (1300) according to anyone of claims 9 to 14.

**Revendications**

1. Un conduit de mesure de pression (105) comprenant une membrane (109) ainsi qu'un connecteur (107) pour relier le conduit de mesure de pression (105) à un appareil de traitement du sang (1000), où le conduit de mesure de pression (105) comprend en outre au moins deux sections de lumière consécutives, notamment la première section de lumière (105a) et la seconde section de lumière (105b), la première section de lumière (105a) comprenant une première géométrie de lumière et la seconde section de lumière (105b) comprenant une seconde géométrie de lumière, où la première géométrie de lumière et la seconde géométrie de lumière diffèrent l'une de l'autre au moins par leur diamètre.

**2.** Le conduit de mesure de pression (105) selon la première revendication, comprenant plusieurs sections de lumière (105a, 105a', 105a") ayant chacune la première géométrie de lumière et/ou plusieurs sections de lumière (105b, 105b', 105b") ayant chacune la seconde géométrie de lumière.

**3.** Le conduit de mesure de pression (105) selon la revendication 2, où des sections de lumière (105a, 105a', 105a") ayant chacune la première géométrie de lumière sont séparées les unes des autres, et/ou où des sections de lumière (105b, 105b', 105b") ayant chacune la seconde géométrie de lumière sont séparées les unes des autres.

**4.** Le conduit de mesure de pression (105) selon l'une quelconque des revendications 2 à 3, où des sections de lumière (105a, 105a', 105a'') ayant chacune la première géométrie de lumière alternent avec des sections de lumière (105b, 105b', 105b'') ayant chacune la seconde géométrie de lumière.

**5.** Le conduit de mesure de pression (105) selon l'une quelconque des revendications précédentes comprenant un connecteur supplémentaire pour relier le conduit de mesure de pression (105) à une chambre à sang (103).

**6.** Une chambre à sang (103) comprenant ou étant reliée à un conduit de mesure de pression (105) selon l'une quelconque des revendications précédentes.

**7.** Un set de tuyaux sanguins extracorporel (100) comprenant ou étant relié à au moins une chambre à sang (103) selon la revendication 6 et/ou au conduit de mesure de pression (105) selon l'une quelconque des revendications 1 à 5.

**8.** Le set de tuyaux sanguins extracorporel (100) selon la revendication 7, où le conduit de mesure de pression (105) est relié à la chambre à sang (103).

**9.** Un dispositif de détection (1300) programmé et/ou configuré pour exécuter ou initier un procédé de surveillance d'un conduit de mesure de pression (105) d'un set de tuyaux sanguins extracorporel (100), le conduit de mesure de pression (105) étant relié à un appareil de traitement du sang (1000), où l'appareil de traitement du sang (1000) comprend un capteur de pression (1007) agencé pour mesurer la pression (P) régnant dans le conduit de mesure de pression (105) et où le conduit de mesure de pression (105) est conçu selon l'une quelconque des revendications 1 à 5; où le procédé comprend les étapes consistant en:

- la mesure, au moyen du capteur de pression (1007), d'une pression (P) régnant dans le conduit de mesure de pression (105) ou de sa variation dans le temps et la détermination d'une valeur d'écart ($\delta^2$) de la pression (P) ou de sa variation; et
- l'évaluation de la valeur d'écart ($\delta^2$) ou d'une variation de la valeur d'écart ($\delta^2$) dans le temps, respectivement au moyen d'une comparaison de la valeur d'écart ($\delta^2$) déterminée, ou de sa variation avec des valeurs, des valeurs seuils, des plages ou des cours préalablement mesurés et/ou sauvegardés à cet effet.

**10.** Le dispositif de détection (1300) selon la revendication 9, où l'évaluation est ou englobe l'obtention d'une conclusion au sujet du conduit de mesure de pression (105).

**11.** Le dispositif de détection (1300) selon l'une quelconque des revendications 9 à 10, où la conclusion obtenue est, ou résulte en ce qu'un risque de mouillage de la membrane (109) et/ou du capteur de pression (1007) existe, qu'une hauteur prédéterminée (h) du niveau de liquide dans le conduit de mesure de pression (105) est atteinte et/ou que le niveau de liquide dans le conduit de mesure de pression (105) augmente.

**12.** Le dispositif de détection (1300) selon l'une quelconque des revendications 9 à 11, où à l'obtention d'une conclusion qu'un risque de mouillage de la membrane (109) existe, un message d'erreur ou une alarme est déclenché(e).

**13.** Le dispositif de détection (1300) selon l'une quelconque des revendications 9 à 12 comprenant l'étape supplémentaire consistant en:

- l'interruption ou l'arrêt d'un traitement du sang au moyen de l'appareil de traitement du sang (1000);
ou comprenant l'étape supplémentaire consistant en:

- la prise de mesures, au moyen de l'appareil de traitement du sang (1000), pour abaisser le niveau de liquide dans la chambre à sang (103);

dans chaque cas, lorsque la conclusion obtenue relative au raccordement est, ou résulte en ce qu'un risque de mouillage et/ou de dépassement d'une hauteur prédéterminée (h) du niveau existe.

**14.** Le dispositif de détection (1300) selon la revendication 9 à 13, comprenant ou étant relié à au moins un dispositif d'affichage et étant configuré pour afficher un résultat de l'exécution du procédé au moyen du dispositif d'affichage.

15. Un appareil de traitement du sang (1000) comprenant ou étant relié à au moins un set de tuyaux sanguins extracorporel (100) selon la revendication 7 ou 8 et/ou à un dispositif de détection (1300) selon l'une quelconque des revendications 9 à 14.

Fig. 1

107

109

1001

105b''

105a''

105b'

105a'

105

105b

105a

103

Fig. 2

Fig. 3a

Fig. 3b

Fig. 4

EP 3 781 231 B1

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011108784 A9
- DE 102015102040 A1